# EUROPÄISCHE PATENTSCHRIFT

(11) **EP 3 704 440 B1**
(45) Veröffentlichungstag und Bekanntmachung des Hinweises auf die Patenterteilung: **22.03.2023**
(21) Anmeldenummer: 19813320.9
(22) Anmeldetag: 02.12.2019
(51) Int. Cl.: G01B 7/31, A61B 5/24, A61B 5/00, A61N 1/08, A61N 1/375

(54) **JUSTIERHILFE, DRAHTLOSE VERBINDERANORDNUNG UND VERFAHREN ZUM ÜBERWACHEN EINER POSITION**
ADJUSTMENT AID, WIRELESS CONNECTOR ASSEMBLY, AND METHOD FOR MONITORING A POSITION
AIDE AU RÉGLAGE, ASSEMBLAGE DE CONNECTEURS SANS FIL ET PROCÉDÉ DE SURVEILLANCE D'UNE POSITION

(30) Priorität: 22.01.2019 DE 102019200740
(43) Veröffentlichungstag der Anmeldung: 09.09.2020
(73) Patentinhaber: Albert-Ludwigs-Universität Freiburg, 79098 Freiburg (DE)
(72) Erfinder: KIELE, Patrick, 79106 Freiburg (DE); KOHLER, Alina, 72074 Tübingen (DE); PASLUOSTA, Cristian, 79279 Vörstetten (DE); STIEGLITZ, Thomas, 79110 Freiburg (DE)
(74) Vertreter: Grünecker Patent- und Rechtsanwälte PartG mbB
(86) Internationale Anmeldenummer: PCT/EP2019/083300
(87) Internationale Veröffentlichungsnummer: WO 2020/151861

(56) Entgegenhaltungen:
- EP-A1- 1 763 078
- EP-A2- 0 240 020
- EP-A2- 2 661 162
- DE-A1-102013 108 733
- US-A- 4 420 754
- US-A1- 2013 079 848

## Beschreibung

Die vorliegende Erfindung betrifft eine Justierhilfe, eine drahtlose elektrische Verbinderanordnung, insbesondere für den Einsatz bei implantierbaren Komponenten, und ein zugehöriges Verfahren zum Überwachen der Position einer ersten Komponente und einer zweiten Komponente mit Bezug aufeinander.

Jüngste Forschung und Entwicklung auf dem Gebiet der Neurotechnik ("Neural Engineering") führten zu einer Vielzahl von aktiven implantierbaren medizinischen Geräten (Active Implantable Medical Device, AIMD), die in einem weiten Anwendungsbereich einsetzbar sind. Diese bestehen üblicherweise aus einem Gehäuse, das eine Steuerelektronik und eine Batterie beinhaltet, implantierbaren Elektroden (oder Elektrodenarrays) und Kabeln für die elektrische Kontaktierung der Elektroden und der Elektronik. Die Elektroden werden für die elektrische Stimulierung von Zellen oder das Erfassen von physiologischen Signalen verwendet.

Reversibel lösbare Verbinder (nachfolgend auch als Konnektoren bezeichnet), die in den Kabeln integriert sind, ermöglichen es, die Einzelteile zu separieren. Somit wird sowohl die Implantation erleichtert als auch die Möglichkeit geschaffen, defekte, verbesserte oder verbrauchte Teile (z. B. die Batterie in einem Herzschrittmacher, die nur eine Lebenszeit von 3 bis 7 Jahren hat) auszutauschen.

Abhängig von der jeweiligen Anwendung werden die Stimulation oder die aufgezeichneten Signale in einer implantierten oder in einer externen Einheit verarbeitet. Perkutane Kabel sind häufig ausreichend für die Energieversorgung und Signalübertragung während akuter Eingriffe. In chronischen Anwendungsfällen vermeidet man dies, da perkutane Kabel ein ernsthaftes Risiko für Entzündungen darstellen. Für chronische Anwendungen ist es vorzuziehen, ein drahtloses transkutanes Übertragungssystem zu verwenden. Beispiele für solche Systeme sind Cochleaimplantate oder der sogenannte Brindley-Stimulator (Blasenschrittmacher), die üblicherweise eine induktive Kopplung sowohl für die Energieversorgung wie auch die Signalübertragung auf einem oder mehreren Kopplungskanälen einsetzen.

Resistive oder kapazitive Kopplung durch die Haut hindurch stellt eine weitere Alternative dar. Beispielsweise ist aus dem Artikel L. S. Gan and A. Prochazka, "Properties of the stimulus router system, a novel neural prosthesis," IEEE transactions on biomedical engineering, vol. 57, no. 2, pp. 450-459, 2010, ein Stimulus Router System (SRS) bekannt, bei dem Stimulationsstrom über selbstklebende Oberflächenelektroden bereitgestellt wird, die auf die Haut geklebt werden. Eine subkutane Empfangselektrode empfängt dieses Signal und leitet es über implantierte Leitungen an den Stimulationsort weiter. Dieses Konzept kann auf Mehrkanalanwendungen erweitert werden, bei denen ein Oberflächenarray von Elektroden kapazitiv mit einem implantierten subkutanen Gegenstück in identischer geometrischer Anordnung gekoppelt wird. Jedes Elektrodenpaar formt dann eine kapazitive gekoppelte Einheit und bildet einen Kanal aus.

Weitere derartige Konzepte sind bekannt aus P. Kiele et al. "Design Rules for a Transcutaneous Capacitive Electrode Array for Functional Electrical Stimulation of Peripheral Nerves", IFESS 2018: A0006, S. 35, Program Book, 2nd Annual Conference ofthe International Functional Electrical Stimulation Society, 28.-31. 08.2018, Notwill, CH; C. Pasluosta et al. "Toward a Multi-Channel Wireless System for Electrical Stimulation of Peripheral Nerve: Modelling and Simulation of Signal Transmission", IFESS 2018: A0011, S. 44, Program Book, 2nd Annual Conference of the International Functional Electrical Stimulation Society, 28.-31. 08.2018, Notwill, CH, sowie P. Kiele et al. "Towards a Capacitive Energy and Signal Supply in Neural Implants: In-vitro Evaluation of Coupling Behavior through Human Skin", Konferenz-Beitrag zu der "Engineering in Medicine and Biology Society Conference" in Hawaii, 2018.

Bei allen diesen Konzepten ist die Ausrichtung der externen Komponente mit Bezug auf die implantierte Komponente essenziell, um ausreichende Genauigkeit und Effizienz bei der Energieversorgung wie auch bei der Energieübertragung sicherzustellen.

Beispielsweise wird in dem Artikel F. Kohler et al., "Closed-loop interaction with the cerebral cortex: A review of wireless implant technology," Brain-Computer Interfaces, vol. 4, no. 3, pp. 146-154, 2017, vorgeschlagen, Magnete für die Ausrichtung der externen Komponente in Bezug auf den implantierten Teil zu verwenden.

EP 2 661 162 A2 offenbart ein Gerät mit einer oder mehreren Einsteckeinheiten. Es wird ein Gerät mit mindestens einer Steckeinheit und einer Grundkörpervorrichtung zur Aufnahme der Steckeinheit vorgestellt. Die Einsteckeinheit umfasst eine elektrisch leitende Struktur mit mindestens einem Oberflächenbereich, der in der Lage ist, eine kapazitive Kopplung mit einem Oberflächenbereich eines elektrisch leitenden Teils der Grundkörpervorrichtung zu bilden, wenn die Einsteckeinheit in die Grundkörpervorrichtung eingesetzt ist. Das Gerät umfasst ferner eine Überwachungsschaltung zum Erzeugen eines Signals, das die elektrischen Eigenschaften eines Messkreises anzeigt, der die kapazitive Kopplung und mindestens einen galvanischen Kontakt umfasst, der von elektrischen Verbindern der Einschubeinheit und der Grundkörpervorrichtung bereitgestellt wird. Das erzeugte Signal ist auch ein Indikator für die Korrektheit der Installation der Steckeinheit. So kann die korrekte Installation der Steckeinheit elektrisch angezeigt und überwacht werden.

EP 1 763 078 A1 bezieht sich auf ein System zur Messung der Ausrichtung zwischen Chips. Insbesondere offenbart diese Druckschrift ein Ausrichtungsmesssystem zum Messen der Ausrichtung zwischen einer Vielzahl von Chips einer zu testenden Vorrichtung, wobei die Chips in einer dreidimensionalen Stapelkonfiguration zusammengebaut und mit mindestens einem integrierten kapazitiven Sensor ausgestattet sind. Das System umfasst eine Mehrfachkondensatorstruktur, die in dem kapazitiven Sensor integriert ist, mindestens eine mit der Mehrfachkondensatorstruktur verbundene Sensorschaltung, die eine Ausgangsspannung ausgibt, die proportional zu einer Änderung eines kapazitiven Wertes der Mehrfachkondensatorstruktur des integrierten kapazitiven Sensors der zu testenden Vorrichtung ist und einem gemessenen Versatz zwischen den Chips der zu testenden Vorrichtung entspricht.

EP 0 240 020 A2 offenbart einen kapazitiven Messwandler zur Positionsmessung. Der kapazitive Wandler zur Positionsmessung weist eine erste Skala und eine zweite Skala auf, die zueinander benachbart derart angeordnet sind, dass sie relativ zueinander bewegt werden können. Beide Skalen sind mit entweder Übertragungselektroden oder Empfangselektroden versehen, von denen beide aus Gruppen von Elektroden bestehen, die in der Richtung angeordnet sind, in der die Skalen relativ bewegbar sind, und kapazitiv miteinander gekoppelt. Die vorgenannten Übertragungselektroden bilden eine Gruppe von Übertragungselektroden, denen eine Anzahl von Wechselstromsignalen unterschiedlicher Phasen zugeführt werden, und die vorgenannten Empfangselektroden bestehen aus einer Anzahl von Elektroden mit gleichbeabstandeter Schrittweite, die durch Teilung der Übertragungswellenlängenschrittweite der vorgenannten Gruppe von Übertragungselektroden durch eine vorbestimmte, ganze Zahl erhalten wird. Eine Skalengenauigkeit von der Größe eines der Anzahl der Übertragungselektroden in der vorgenannten Gruppe von Übertragungselektroden entsprechenden Bruchteils der Empfangselektrodenschrittweite ist dadurch möglich.

Nachteilig ist bei den bekannten Anordnungen jedoch, dass die Stimulations- bzw. Aufzeichnungsvorrichtungen über keine Möglichkeit einer Echtzeitüberwachung verfügen, mit der eventuelle Fehlausrichtungen korrigiert werden könnten. Für Mehrkanalanwendungen ist eine solche Überwachung aber von entscheidender Bedeutung. Während bei induktiven Systemen Fehlausrichtungen zwar dadurch kompensiert werden können, dass größere externe Spulen verwendet werden, ist diese Vorgehensweise oft durch die Miniaturisierung des Implantats und ein zunehmendes Risiko von Übersprechen zwischen den Kanälen limitiert.

Es besteht daher ein Bedarf, eine aktive Überwachung der Ausrichtung einer externen Komponente mit Bezug auf eine implantierte Einheit bereitzustellen, um fehlerfreies Funktionieren des Implantats sicherzustellen, insbesondere wenn ein verteiltes mehrkanaliges Array verwendet wird.

Die vorliegende Erfindung stellt sich die Aufgabe, eine Justierhilfe zum Ausrichten mindestens einer ersten und einer zweiten Komponente mit Bezug aufeinander anzugeben, die es erlaubt, auch dicht gepackte Kontaktarrays zuverlässig und passgenau zu verbinden. Dabei soll die Justierhilfe möglichst kostengünstig und darüber hinaus biokompatibel und zertifizierbar für den chronischen Einsatz sein. Eine Langzeitüberwachung der korrekten Ausrichtung sollte darüber hinaus möglich sein.

Diese Aufgaben werden durch den Gegenstand der unabhängigen Patentansprüche gelöst. Vorteilhafte Weiterbildungen der vorliegenden Erfindung sind Gegenstand mehrerer abhängiger Patentansprüche.

Die vorliegende Erfindung basiert auf der Idee, dass ein externes Anregungssignal an eine erste vorzugsweise außerhalb angeordnete Elektrode angelegt wird. Das Signal wird anschließend an eine beispielsweise implantierte Gegenelektrode eingekoppelt, von wo es über eine elektrische Verbindung an mindestens eine weitere Elektrode übertragen wird. Von dort wird das Anregungssignal zurück an die externe Einheit ausgekoppelt und die elektrische Antwort wird gemessen. Wenn eine Fehlausrichtung der externen Einheit mit Bezug auf das korrespondierende, beispielsweise implantierte Array vorliegt, nehmen die Kopplungsimpedanzen zu und die detektierbare elektrische Antwort fällt ab.

Insbesondere stellt die vorliegende Erfindung eine Justierhilfe zum Ausrichten mindestens einer ersten und einer zweiten Komponente mit Bezug aufeinander bereit. Die Justierhilfe weist eine erste Justierelektrodenanordnung auf, die mindestens eine Anregungselektrode und mindestens eine Auswerteelektrode aufweist und an der ersten Komponente anordenbar ist. Eine zweite Justierelektrodenanordnung ist vorgesehen, die mindestens eine Empfangselektrode und mindestens eine Sendeelektrode aufweist und an der zweiten Komponente anordenbar ist. Eine Steuer- und Auswerteschaltung ist mit der ersten Justierelektrodenanordnung verbunden, wobei die Steuer- und Auswerteschaltung betrieben werden kann, die Anregungselektrode mit einer Anregungsspannung zu speisen und an der Auswerteelektrode ein Messsignal abzugreifen, das von einem Überdeckungsgrad der ersten und zweiten Justierelektrodenanordnung abhängt.

Die Anregungselektrode kann (abwechselnd) ebenfalls als Auswerteelektrode und dementsprechend das implantierbare Pendant sowohl als Empfangs- als auch als Sendeelektrode fungieren. So können beispielsweise zeitlich oder räumlich auftretende Inhomogenitäten kompensiert werden. Beispielsweise regt Elektrode 1 zum Zeitpunkt t1 an, während Elektroden 2 und 3 auswerten. Zum Zeitpunkt t2 regt Elektrode 2 an, während die Elektroden 1 und 3 auswerten, usw.

In vorteilhafter Weise kann auf diese Weise kontinuierlich überwacht werden, ob die erste und zweite Komponente mit Bezug aufeinander optimal justiert sind, auch wenn die zweite Komponente nicht mehr ohne weiteres zugänglich ist. Darüber hinaus hat die erfindungsgemäße Anordnung den Vorteil, dass sie einfach und kostengünstig herstellbar ist und insbesondere die Elektrodenanordnungen ohne Zusatzaufwand zusammen mit den ohnehin vorhandenen elektrisch leitfähigen Strukturen der ersten und zweiten Komponenten hergestellt werden können. Darüber hinaus beanspruchen die Justierelektrodenanordnungen nur sehr wenig Platz. Dieser Vorteil kommt insbesondere bei implantierten Anordnungen zum Tragen.

Mit der Anordnung gemäß der vorliegenden Erfindung kann eine einfach zu integrierende aktive Überwachung der Ausrichtung zweier Komponenten zueinander bereitgestellt werden, wobei eine der beiden Komponenten vollständig elektrisch passiv sein kann. Da das Eingangssignal und das gemessene Ausgangssignal nur an einer der beiden Komponenten angelegt bzw. abgegriffen werden, kann die Justierhilfe auch an schwer zugänglichen Stellen genutzt werden, oder wenn die dazwischenliegende Schicht (z. B. die Haut) nicht beschädigt werden darf. Schließlich kann die Justierhilfe gemäß der vorliegenden Erfindung zusätzlich in bestehende System integriert werden.

Da keine ferromagnetischen Materialien für die Justierung benötigt werden, besteht die Möglichkeit, die Justiereinrichtung auch für Magnetresonanzuntersuchungen (MRI) sicher auszulegen und einzusetzen.

Gemäß einer vorteilhaften Ausführungsform der vorliegenden Erfindung sind im optimal justierten Zustand der ersten und zweiten Komponente mit Bezug aufeinander die Anregungselektrode und die Empfangselektrode einander überdeckend ausgerichtet, um einen Speisekondensator auszubilden, und die Sendeelektrode und die Auswerteelektrode sind einander überdeckend ausgerichtet, um einen Messkondensator auszubilden. Eine solche kapazitive Kopplung hat den Vorteil, dass kein ohmscher Kontakt benötigt wird.

Im Gegensatz zu ohmschen Kontakten müssen die elektrisch leitfähigen Kopplungselektroden nicht geöffnet werden, d. h. eine isolierende Materialschicht kann darauf verbleiben. Diese kann z. B. aus Polymeren (Parylene-C, PDMS), Oxiden (TiOx) oder anderen Materialien bestehen. Parylene-C ist beispielsweise ein für Humanimplantationen zugelassenes Material und ist bei einer Dicke von 10 µm elektrisch dicht. Diverse Oxide erreichen diese elektrische Dichtigkeit bereits bei deutlich niedrigeren Schichtdicken und weisen eine höhere Dielektrizitätszahl auf (z.B. ε_{r,TiO2} = 63,7; ε_{r,parylene-C} = 3,1). Beide Faktoren versprechen eine Steigerung der Kopplungskapazität und dementsprechend eine bessere Kopplung. Wird keine Isolierung verwendet, kann die elektrische Kopplung kapazitiv und resistiv über eingelagertes Wasser erfolgen. In diesem Fall müssen allerdings benachbarte Kontakte gegeneinander isoliert werden. Generell wählt man als Isolatormaterial für die Anwendung in implantierbaren Komponenten üblicherweise Silikonkautschuk (Polydimethylsiloxan, PDMS). PDMS hat die ausreichende Langzeitstabilität, die für den jahrelangen Gebrauch in wässriger oder feuchter Umgebung, wie sie für eine aktive implantierbare Baugruppe auftritt, gefordert werden muss. Jedes andere geeignete Material kann selbstverständlich auch verwendet werden.

Gemäß einer vorteilhaften Ausführungsform der vorliegenden Erfindung sind die mindestens eine Empfangselektrode und die mindestens eine Sendeelektrode elektrisch leitend miteinander verbunden. Auf diese Weise kann der Messkreis in besonders einfacher Weise geschlossen werden. Selbstverständlich können die die mindestens eine Empfangselektrode und die mindestens eine Sendeelektrode auch auf andere Weise, beispielsweise kapazitiv über Kammstrukturen oder induktiv gekoppelt sein.

Eine besonders einfache und kostengünstige Herstellbarkeit kann erreicht werden, wenn die erste Justierelektrodenanordnung ein erstes planares Elektrodenarray aufweist, und die zweite Justierelektrodenanordnung ein zweites planares Elektrodenarray aufweist, das in einer Ebene parallel zu dem ersten planaren Elektrodenarray angeordnet ist, wenn die erste und zweite Komponente mit Bezug aufeinander justiert sind. Mit dieser Geometrie ist die Justierhilfe kompatibel zu planaren Konnektoren, wie sie zum elektrischen Kontaktieren von AIMD häufig verwendet werden.

Gemäß einer vorteilhaften Weiterbildung der vorliegenden Erfindung weist die erste Justierelektrodenanordnung zwei Auswerteelektroden auf, die so angeordnet sind, dass ihre (gedachten) Verbindungslinien mit der Anregungselektrode einander unter einem Winkel verschieden von 0° schneiden, und wobei die zweite Justierelektrodenanordnung zwei Sendeelektroden aufweist, die so angeordnet sind, dass ihre Verbindungslinien mit der Empfangselektrode einander unter einem Winkel verschieden von 0° schneiden. Mit einer solchen Anordnung kann in vorteilhafter Weise sowohl eine lineare wie auch eine Winkelabweichung von der idealen Position detektiert werden. In besonders vorteilhafter Weise schneiden sich die beiden gedachten Verbindungsachsen unter 90°, so dass eine L-förmige Elektrodengeometrie ausgebildet ist. Diese Anordnung hat den Vorteil, dass die Berechnung von X- und Y-Komponenten der Positionsabweichungen besonders einfach berechnet werden kann. Bei geeigneter Kalibrierung und Verwendung entsprechender Auswertealgorithmen sind andere Winkel natürlich ebenfalls möglich, falls dies beispielsweise aus Raumgründen erforderlich ist.

Eine verbesserte Genauigkeit insbesondere bei der Auswertung von Abweichungen durch Verdrehen erreicht man, indem eine dritte Auswerteelektrode vorgesehen wird, die auf der (gedachten) Verbindungslinie der zweiten Auswerteelektrode und der Anregungselektrode angeordnet ist, wobei die zweite Justierelektrodenanordnung eine korrespondierende dritte Sendeelektrode aufweist, die auf der Verbindungslinie zwischen der zweiten Sendeelektrode und der Empfangselektrode angeordnet ist. Dabei ist die dritte Auswerteelektrode vorzugsweise nicht zwischen der der zweiten Auswerteelektrode und der Anregungselektrode angeordnet, sondern außerhalb. Selbstverständlich ist dies aber lediglich eine Definitionsfrage.

Um die Berechnungen zu vereinfachen, können die Anregungselektrode und die Auswerteelektroden sowie die Empfangselektrode und die Sendeelektroden äquidistant angeordnet sein.

Die Elektrodenform kann gemäß der vorliegenden Erfindung beliebig gewählt werden. Vorzugsweise haben die Anregungselektrode und die Auswerteelektroden sowie die Empfangselektrode und die Sendeelektrode kreisförmige und/oder elliptische und/oder polygonale Metallisierungsstrukturen. Diese können auf besonders einfache Weise z. B. durch Fotostrukturierung oder Laserstrukturierung herstellen.

Die Vorteile der erfindungsgemäßen Justierhilfe lassen sich besonders effizient bei einer drahtlosen Verbindereinheit zu einem implantierten Gerät nutzen. Daher bezieht sich die vorliegende Erfindung weiterhin auf eine drahtlose Verbinderanordnung mit einer ersten und einer zweiten Komponente und mit einer Justierhilfe nach einem der vorhergehenden Ansprüche, wobei mindestens die zweite Komponente implantierbar ist, und wobei die erste Komponente eine erste Justierelektrodenanordnung und die zweite Komponente eine zweite Justierelektrodenanordnung trägt.

Insbesondere ist die Justierhilfe vorteilhaft einsetzbar, wenn die erste Komponente ein implantierbares Kopplungselektrodenarray aufweist, und die zweite Komponente ein externes Kopplungselektrodenarray aufweist, welches das implantierbare Kopplungselektrodenarray im implantierten Zustand elektrisch kontaktiert. Derartige Kopplungselektrodenarrays können beispielsweise ebenfalls auf dem kapazitiven Prinzip arbeiten und sind z. B. in der deutschen Patentanmeldung DE 10 2018 219 831 A1 beschrieben.

Gemäß einer vorteilhaften Ausführungsform kann die Justierhilfe gemäß der vorliegenden Erfindung auch mit Koaxialverbindern verwendet werden, z. B. mit einem Herzschrittmacher, aber auch mit jeglichen anderen implantierbaren Systemen, die eine Stimulationselektrode (ohne Recording) aufweisen und ansteuern, wie beispielsweise Tiefenhirnstimulatoren, Vagusnervstimulatoren, peripheren Nervenstimulatoren usw., wobei die erste Komponente einen implantierbaren Koaxialsteckverbinder aufweist und die zweite Komponente einen implantierbaren Koaxialgegensteckverbinder aufweist. Beispielsweise ist die erste Komponente eine Koaxialbuchse eines Herzschrittmachers und die zweite Komponente der Koaxialstecker einer Stimulationselektrode. Bei Herzschrittmachern besteht üblicherweise keine Möglichkeit, zu kontrollieren, ob die Stimulationselektrode auch tatsächlich mit dem Header des eigentlichen Geräts verbunden ist. Die Tatsache, dass sich der Verbinder der Stimulationselektrode aus dem Header gelöst hat, führt aber zum Ausfall der Stimulation und damit zum völligen (lebensgefährlichen) Versagen des Schrittmachers. Mit der erfindungsgemäßen Anordnung kann eine Fehljustierung zwischen Header und Stimulationselektrodenverbinder bereits detektiert werden, wenn noch Stimulationssignale an die Stimulationselektrode übertragen werden, und es können rechtzeitig entsprechende Gegenmaßnahmen eingeleitet werden.

Die vorliegende Erfindung bezieht sich weiterhin auf ein Verfahren zum Überwachen der Position einer ersten Komponente und einer zweiten Komponente mit Bezug aufeinander, wobei das Verfahren eine Justierhilfe gemäß der vorliegenden Erfindung verwendet und die folgenden Schritte aufweist:
Anlegen einer Anregungsspannung an die mindestens eine Anregungselektrode,
Abgreifen einer Messspannung an der mindestens einen Auswerteelektrode,
Vergleichen der Messspannung mit einem voreingestellten Schwellenwert.

Insbesondere kann beim Feststellen einer Fehljustierung das Verfahren weiterhin vorsehen, dass die Position der ersten Komponente und der zweiten Komponente mit Bezug aufeinander solange verändert wird, bis die Messspannung den voreingestellten Schwellenwert übersteigt.

Alternativ oder zusätzlich kann auch vorgesehen sein, dass die Steuer- und Auswerteschaltung ein Warnsignal erzeugt, wenn die Messspannung unterhalb des voreingestellten Schwellenwerts liegt. Eine solche Warnmeldung ist besonders vorteilhaft bei einer Verwendung mit einem chronisch implantierten Gerät wie einem Herzschrittmacher.

Zum besseren Verständnis der vorliegenden Erfindung wird diese anhand der in den nachfolgenden Figuren dargestellten Ausführungsbeispiele näher erläutert. Dabei werden gleiche Teile mit gleichen Bezugszeichen und gleichen Bauteilbezeichnungen versehen. Weiterhin können auch einige Merkmale oder Merkmalskombinationen aus den gezeigten und beschriebenen unterschiedlichen Ausführungsformen für sich eigenständige, erfinderische oder erfindungsgemäße Lösungen darstellen. Dabei zeigen:
- **FIG. 1**: eine schematische perspektivische Prinzipdarstellung einer Justierhilfe gemäß einer ersten vorteilhaften Ausführungsform der vorliegenden Erfindung;
- **FIG. 2**: eine schematische Draufsicht einer kapazitiven Verbinderanordnung mit einer Justierelektrodenanordnung gemäß einer weiteren vorteilhaften Ausführungsform;
- **FIG. 3**: eine schematische Draufsicht einer kapazitiven Verbinderanordnung mit einer Justierelektrodenanordnung gemäß einer weiteren vorteilhaften Ausführungsform;
- **FIG. 4**: eine graphische Darstellung der Messsignale im Falle einer linearen Fehlausrichtung;
- **FIG. 5**: eine graphische Darstellung der Messsignale im Falle einer rotatorischen Fehlausrichtung;
- **FIG. 6**: eine schematische Darstellung einer Justierelektrodenanordnung gemäß einer weiteren vorteilhaften Ausführungsform bei linearer Fehlausrichtung;
- **FIG. 7**: eine schematische Darstellung einer Justierelektrodenanordnung gemäß einer weiteren vorteilhaften Ausführungsform bei rotatorischer Fehlausrichtung;
- **FIG. 8**: eine schematische perspektivische Darstellung eines Headers mit einer Justierhilfe gemäß einer weiteren vorteilhaften Ausführungsform der vorliegenden Erfindung.

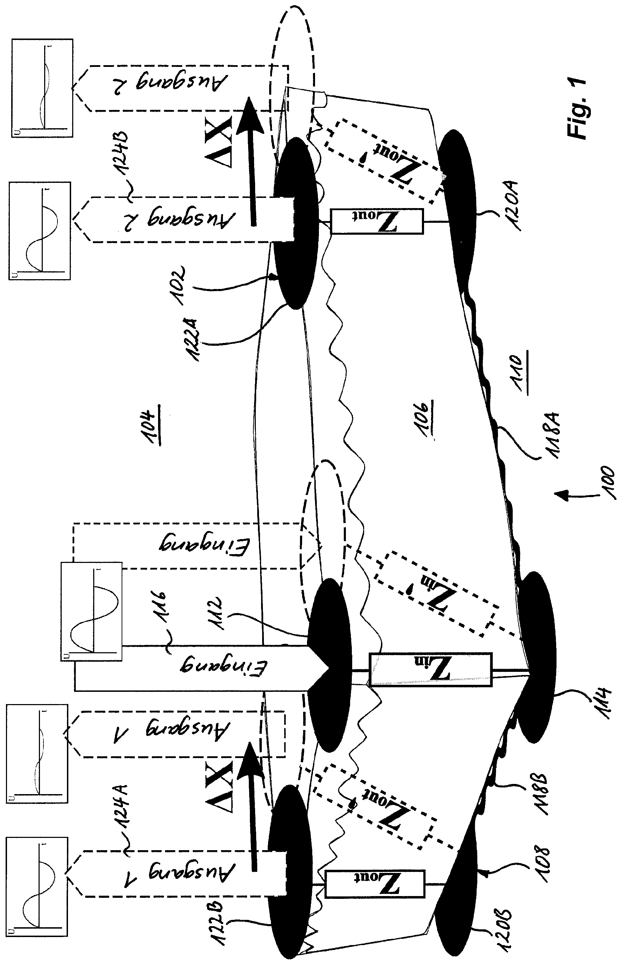

Mit Bezug auf die Figur 1 soll nachfolgend zunächst das Prinzip der Justierhilfe 100 gemäß einer ersten vorteilhaften Ausführungsform der vorliegenden Erfindung näher erläutert werden. Figur 1 zeigt eine perspektivische Ansicht Justierhilfe 100 im verbundenen Zustand.

Dabei befindet sich eine erste Justierelektrodenanordnung 102 auf einer Außenseite 104 der Haut 106. Eine zweite Justierelektrodenanordnung 108 ist an der Innenseite 110 der Haut 106 angeordnet. Beispielsweise können die erste Justierelektrodenanordnung 102 und die zweite Justierelektrodenanordnung 108 Teile eines Verbinders sein, der durch die Haut hindurch ein aktives implantiertes medizinisches System (AIMD) elektrisch kontaktiert und mit Energie versorgt. Für einen Fachmann ist jedoch klar, dass die Prinzipien der vorliegenden Erfindung auch für andere Anwendungsfälle eingesetzt werden können, bei denen zwei Teile, die mit Bezug aufeinander beweglich sind, exakt zueinander justiert werden müssen und eines der beiden Teile nach dem Anbringen der Justierelektrodenanordnung nicht mehr ohne weiteres zugänglich ist.

In Figur 1 ist die zweite Justierelektrodenanordnung 108 ortsfest im Innenbereich 110 unter der Haut 106 implantiert, während die erste Justierelektrodenanordnung 102, wie durch die gestrichelten Elemente symbolisiert, verschieblich ist.

Gemäß der gezeigten Ausführungsform umfasst die implantierte zweite Justierelektrodenanordnung 108 drei elektrisch miteinander verbundene Elektroden, die eine ähnliche Struktur wie die erste Justierelektrodenanordnung 102 ausbilden.

Die erste Justierelektrodenanordnung 102 weist drei korrespondierende nicht miteinander verbundene Elektroden an der externen Einheit auf.

Erfindungsgemäß ist eine Anregungselektrode 112 vorgesehen, die ein Anregungssignal 116 an die korrespondierende Empfangselektrode 114 ausgibt. Das Anregungssignal 116 wird kapazitiv durch die Haut 106 hindurch an die implantierte Gegenelektrode 114 übertragen. Über elektrische Verbindungen 118A, 118B wird das Anregungssignal an zwei Sendeelektroden 120A, 120B übertragen. Von dort wird, wiederum kapazitiv, ein Signal an zwei korrespondierende, im Außenbereich 104 angeordnete Auswerteelektroden 122A, 122B ausgekoppelt. Die elektrische Antwort, die an den Auswerteelektroden 122 abgegriffen werden kann, bildet ein Messsignal 124.

Sofern eine Fehljustierung der im Außenbereich 104 befindlichen Komponente mit Bezug auf die implantierte Komponente vorliegt, erhöht sich die Kopplungsimpedanz Z sowohl zwischen den Anregungs- und Empfangselektroden (Zᵢₙ) wie auch zwischen den Sende- und Auswerteelektroden (Zₒᵤₜ). Diese Impedanzerhöhungen führen zu einer Verringerung der elektrischen Antwort. Durch Auswerten der Messsignale 124A, 124B kann auf eine Verschiebung ΔX geschlossen werden. Bei entsprechender Kalibrierung ist es auch möglich, quantitative Aussagen über die Verschiebung ΔX zu treffen.

Wie mit Bezug auf die nachfolgenden Figuren noch deutlich wird, kann die Justierhilfe 100 selbstverständlich auch andere Elektrodengeometrien als die in Figur 1 gezeigte aufweisen. Jegliche Anordnungen mit mehr als zwei Elektrodenpaaren ist möglich. Zwei Elektrodenpaare müssen mindestens vorgesehen sein, es können aber auch mehr als zwei verwendet werden, um mehrere Freiheitsgrade gleichzeitig zu überwachen. In vorteilhafter Weise sind die jeweils korrespondierenden Elektroden von gleicher Größe und Form. Selbstverständlich ist dies aber nicht zwingend nötig, da nur jeweils der Überlapp der einander gegenüberliegenden Elektroden in das Messsignal eingeht und durch entsprechende Kalibrierungsschritte das gewünschte Messsignal im Falle einer optimalen Justierung festgelegt werden kann.

Erfindungsgemäß kann die Einhaltung der optimalen Justierung überwacht werden, indem die elektrische Antwort auf zwei Übertragungskanälen gemessen wird. Die elektrische Antwort ist dabei proportional zu den Änderungen in der Kopplungsimpedanz. Beispielsweise können Frequenzen von mehr als 1 kHz und niedrige Spannungen im Bereich von ca. 1,2 V verwendet werden, um zu verhindern, dass Nervenbahnen oder Rezeptoren, die in der Haut 106 liegen, angeregt werden. Die Justierhilfe 100 gemäß der vorliegenden Erfindung kann leicht in der Nähe einer Stimulations- oder Aufzeichnungselektrode angebracht werden. Zusätzlich bietet die vorliegende Konfiguration die Möglichkeit, eine simultane Überwachung von linearen Verschiebungen innerhalb einer Ebene wie auch von rotatorischen Abweichungen um jede beliebige Achse durchzuführen.

Darüber hinaus kann je nach Anwendung anstelle eines kapazitiven Kopplungskonzepts auch ein anderes physikalisches Konzept (zum Beispiel resistive oder induktive Kopplung) genutzt werden. Beispielsweise können auch Elektroden, die planare Spulenanordnungen bilden, zusätzlich zu oder anstelle von kapazitiven Elektroden verwendet werden.

Die Kopplungselektroden können durch Legierungen wie Platin-Iridium oder MP35N^{®} gebildet sein. MP35N^{®} ist ein eingetragenes Warenzeichen von Standard Pressed Steel Technologies, Inc. Die Bestandteile von MP35N^{®} sorgen für eine ausgezeichnete Korrosionsbeständigkeit der Legierung, da alle vier Legierungsbestandteile Nickel, Kobalt, Chrom und Molybdän die Korrosionsbeständigkeit in fast allen in der Industrie gebräuchlichen Edelstahl-, Nickel- und Kobaltlegierungen steigern. Der Anteil von 20% Chrom verbessert die Beständigkeit gegen Oxidation, Sulfidierung und chemische Reaktionen mit Salz bei erhöhten Temperaturen. Jedes andere elektrisch leitfähige Elektrodenmaterial kann selbstverständlich ebenfalls verwendet werden. Für MRI Untersuchungen sollten allerdings nur Materialien verwendet werden, die nicht ferromagnetisch sind.

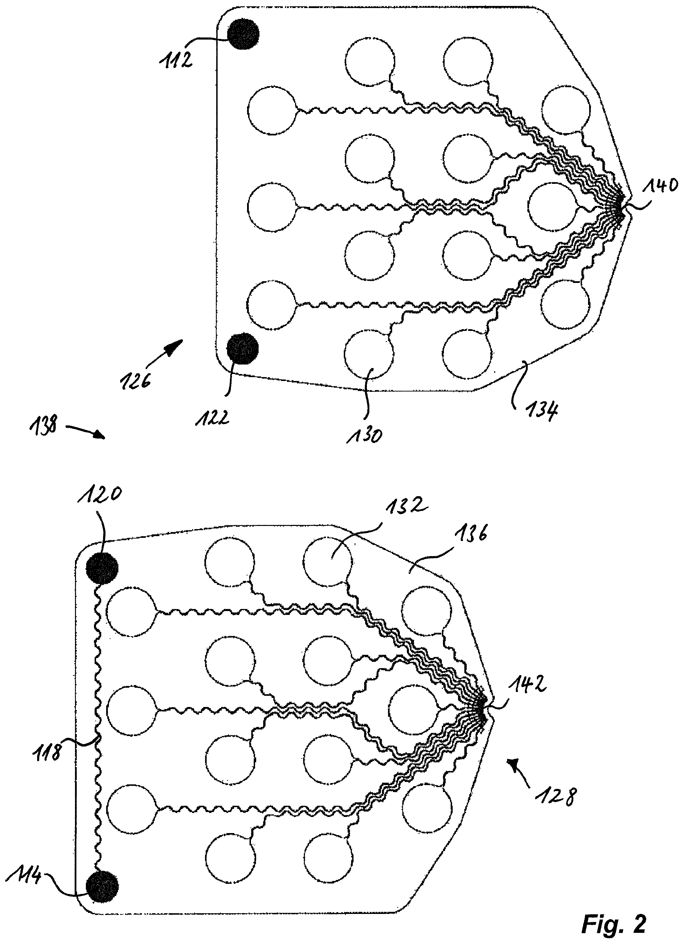

Figur 2 zeigt einen implantierbaren kapazitiv koppelnden Verbinder 128, der beispielsweise unter der Haut implantiert werden kann und die Verbindung zu einer mehrkanaligen Stimulationselektrodenanordnung oder einem anderen AIMD (nicht in den Figuren gezeigt) herstellen kann. Ein komplementärer Gegenverbinder 126 wird im Betrieb so in unmittelbarer Nähe des Verbinders 128 angeordnet, dass die Vielzahl von ersten Kopplungselektroden 130 mit der entsprechenden Vielzahl von zweiten Kopplungselektroden 132 eine kapazitive gekoppelte Signalübertragung ermöglichen. Insbesondere bei einer großen Anzahl von miteinander zu koppelnd Verbindungspunkten ist es von essenzieller Bedeutung zu überwachen ob der Verbinder 128 noch optimal ausgerichtet ist mit Bezug auf den Gegenverbinder 126.

Zu diesem Zweck ist gemäß der vorliegenden Erfindung eine Justierhilfe vorgesehen, die in der in Figur 2 gezeigten Ausführungsform auf ihre einfachsten Bestandteile reduziert ist. Der implantierbare Verbinder 128 weist eine Empfangselektrode 114 und eine Sendeelektrode 120 auf. Die Empfangselektrode 114 und die Sendeelektrode 120 sind miteinander über eine elektrisch leitende Verbindung 118, beispielsweise eine metallisierte Leiterbahn, verbunden. Die zugehörige erste Justierelektrodenanordnung, die auf dem Verbinder 126 angeordnet ist, umfasst eine Anregungselektrode 112 und eine Auswerteelektrode 122.

Weiterhin ist eine Steuer- und Auswerteschaltung (in den Figuren nicht sichtbar) vorgesehen, um die Justierung der beiden Verbinder 126, 128 mit Bezug aufeinander zu überwachen. Hierzu wird, wie bereits oben bezüglich der ersten Ausführungsform erläutert, ein Anregungssignal an die Anregungselektrode 112 angelegt. Dieses wird kapazitiv durch die Haut hindurch von der Empfangselektrode 114 aufgenommen und über die elektrische Verbindung 118 an die Sendeelektrode 120 weitergeleitet. Auf kapazitivem Wege gelangt das Signal wiederum durch die Haut hindurch nach außen an die Auswerteelektrode 122. Sobald der erste und der zweite Verbinder 126, 128 nicht mehr ausreichend gut ausgerichtet sind, verlieren einer oder beide der kapazitiven Kopplungswege ganz oder teilweise den Kontakt und an der Auswerteelektrode 122 kann nur noch ein abgeschwächtes oder gar kein Messsignal mehr empfangen werden.

Wie bereits erwähnt, können die Elektroden durch Legierungen wie Platin-Iridium oder MP35N^{®} gebildet sein. Zur elektrischen Isolierung und zum Schutz gegen die äußere Umgebung ist eine elektrisch isolierende Passivierungsschicht vorgesehen. In vorteilhafter Weise weist der erste Verbinder 126 und/oder der zweite Verbinder 128 ein elektrisch isolierendes Substrat 134 auf. Das Substratmaterial beider Verbinder kann je nach Ausführung flexibel oder starr sein, sollte aber nicht elektrisch leitend sein. Bei elektrisch leitendem Material müssen zusätzliche Isolationsschichten eingebracht werden. Es eignen sich beispielsweise Polyimid, PDMS, Keramik sowie alle anderen Materialien, die für elektrische Schaltungsträger eingesetzt werden.

Die Kopplungselektroden 130, 132 enden in Anschlussbereichen 140, 142, an die eine externe Schaltung bzw. eine Stimulationselektrode angeschlossen werden können.

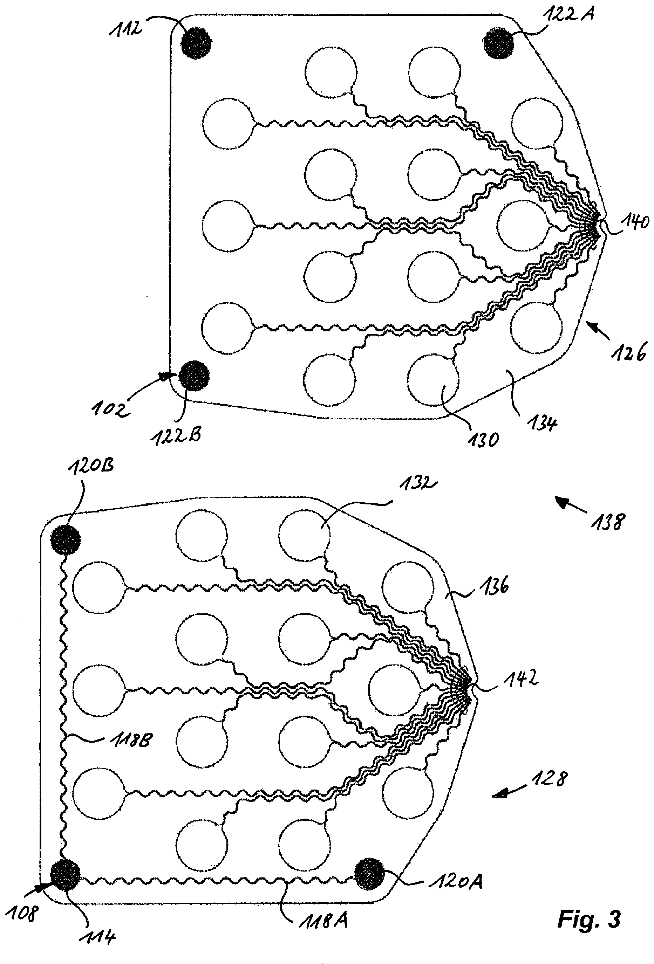

In Figur 3 ist die Verbinderanordnung 138 mit einer weiteren Ausgestaltung der Justierhilfe gezeigt, die im Wesentlichen der L-förmigen Anordnung aus Fig. 1 entspricht.

Gemäß der gezeigten Ausführungsform umfasst die implantierte zweite Justierelektrodenanordnung 108 drei elektrisch miteinander verbundene Elektroden, die eine L-förmige Struktur ausbilden. Die erste Justierelektrodenanordnung 102 weist drei korrespondierende nicht miteinander verbundene Elektroden an der externen Einheit 126 auf.

Erfindungsgemäß ist eine Anregungselektrode 112 vorgesehen, die ein Anregungssignal 116 an die korrespondierende Empfangselektrode 114 ausgibt. Das Anregungssignal 116 wird kapazitiv durch die Haut 106 hindurch an die implantierte Gegenelektrode 114 übertragen. Über elektrische Verbindungen 118A, 118B wird das Anregungssignal an zwei Sendeelektroden 120A, 120B übertragen. Von dort wird, wiederum kapazitiv, ein Signal an zwei korrespondierende, an dem Verbinder 126 angeordnete Auswerteelektroden 122A, 122B ausgekoppelt. Die elektrische Antwort, die an den Auswerteelektroden 122 abgegriffen werden kann, bildet ein Messsignal.

Sofern eine Fehljustierung des im Außenbereich 104 befindlichen Verbinders 126 mit Bezug auf die implantierte Komponente 128 vorliegt, erhöht sich die Kopplungsimpedanz sowohl zwischen den Anregungs- und Empfangselektroden wie auch zwischen den Sende- und Auswerteelektroden. Diese Impedanzerhöhungen führen zu einer Verringerung der elektrischen Antwort. Durch Auswerten der Messsignale, die an den Auswerteelektroden 122A, 122B abgegriffen werden können, kann auf eine Verschiebung oder Verdrehung geschlossen werden. Bei entsprechender Kalibrierung ist es auch möglich, quantitative Aussagen über die Fehljustierung zwischen den beiden Verbindern 126, 128 zu treffen.

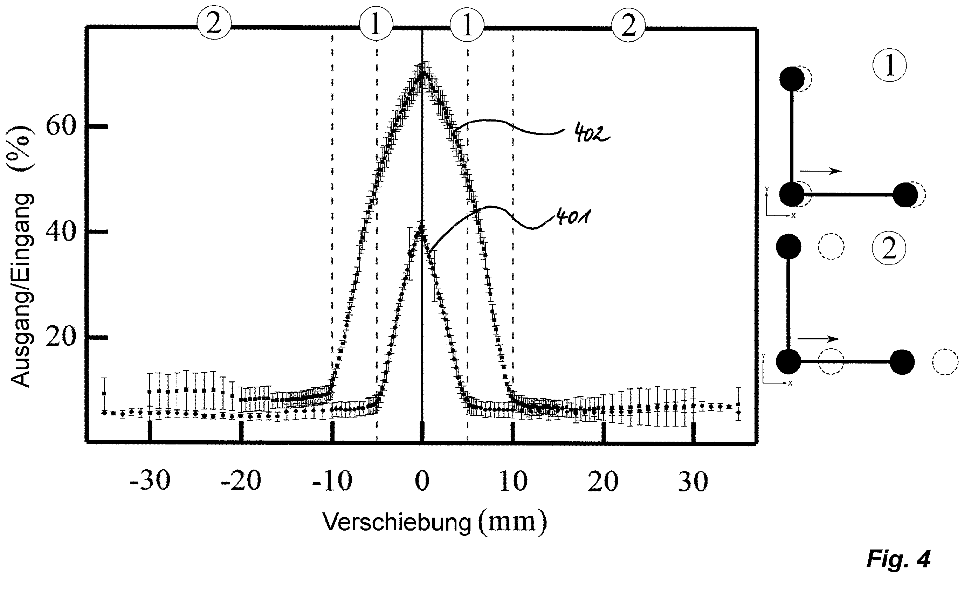

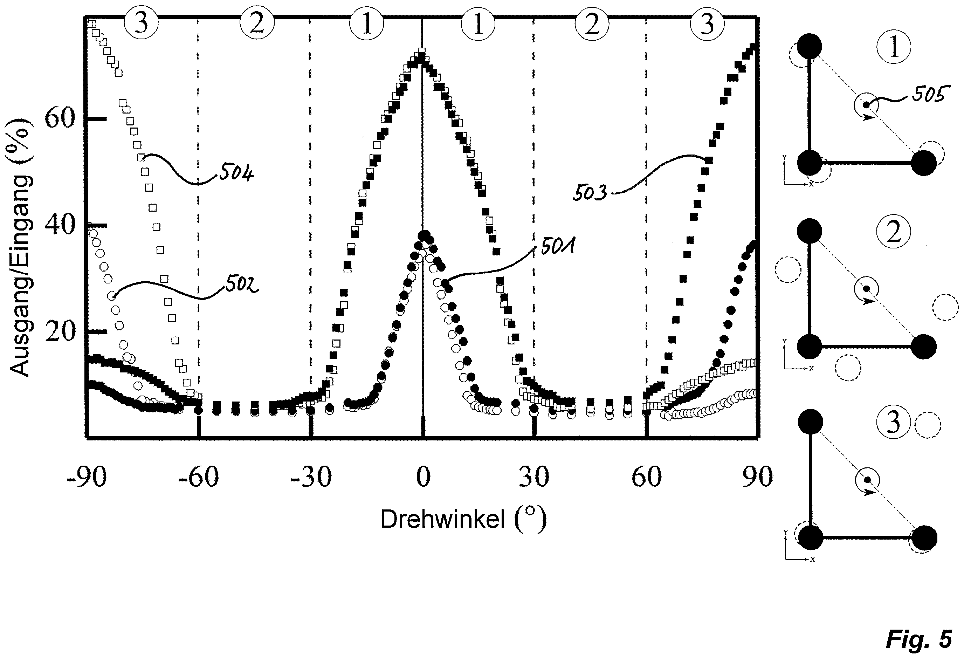

Die Figuren 4 und 5 zeigen Messergebnisse, die mit der L-förmigen Anordnung aus den Figuren 1 und 3 unter Verwendung von hochreinem Wasser als Modell für die Haut erhalten werden. Betrachtet man zunächst Fig. 4, so ist darin das Verhältnis des gemessenen Ausgangssignals zum eingespeisten Anregungssignal (Ausgang/Eingang) als Funktion einer linearen Verschiebung aufgetragen. Dabei zeigt die Kurve 401 Messergebnisse für eine Justierhilfe, bei der die Elektroden eine kreisförmige Gestalt mit einem Durchmesser von 5 mm haben. Die Kurve 402 zeigt die entsprechenden Ergebnisse für kreisförmige Elektroden mit einem Durchmesser von 10 mm.

Man erkennt, dass beide Kurven 401, 402 ihr Maximum bei einer optimalen Ausrichtung (d. h. keine Verschiebung zwischen der ersten und der zweiten Justierelektrodenanordnung und mithin maximale Überdeckung) haben. Die Verluste sind bei größerer Elektrodenfläche geringer, so dass ein höheres Ausgangssignal gemessen werden kann, d. h. das Maximum liegt näher bei dem Idealwert 1. Das Verhältnis sinkt mit zunehmender Verschiebung zwischen der ersten und der zweiten Justierelektrodenanordnung (Bereich ①).

Weiterhin fallen beide Kurven 401, 402 jeweils dann auf den minimalen Basiswert, wenn die Überdeckung zwischen der ersten und der zweiten Justierelektrodenanordnung nicht mehr vorhanden ist (Bereich ②). Der konstante Basiswert ist auf resistive Leckpfade zurückzuführen.

Das gezeigte Verhalten weist außerdem eine Rotationssymmetrie auf.

Fig. 5 illustriert die Ergebnisse bei einer reinen Rotation um einen Drehpunkt 505, der in der Mitte der Hypotenuse des gleichschenkligen Dreiecks liegt, das durch die L-förmig angeordneten Elektroden gebildet ist. Dabei bezeichnen die Kurven 501 und 502 jeweils separat die Signale der Auswerteelektroden für eine Justierhilfe, bei der die Elektroden eine kreisförmige Gestalt mit einem Durchmesser von 5 mm haben. Die Kurven 503 und 504 zeigt die entsprechenden Ergebnisse für kreisförmige Elektroden mit einem Durchmesser von 10 mm. Allerdings können die Elektroden auch rechteckförmig, oval, dreieckförmig oder mit jeder anderen geeigneten Umrissform ausgestaltet sein.

Wie schematisch neben der Messgraphik angedeutet, können drei Bereiche unterschieden werden. Wiederum ist bei einem Drehwinkel von 0° das jeweilige maximale Signal erreicht. Im Bereich ① überdecken sich alle Elektroden. Mit zunehmendem Drehwinkel reduziert sich diese Überdeckung und das Signal sinkt (Bereich ①). In dem Bereich ② überlappen sich keine Elektrodenpaare, so dass hier der im wesentlichen konstante Basiswert gemessen werden kann. In dem Bereich ③ schließlich überlappt sich die Anregungselektrode mit einer der Randelektroden, so dass ein deutlicher Anstieg des Signals zu verzeichnen ist. Dieser zweite Spitzenwert hat dieselbe Größe wie ein Einzelkanal im Falle der vollständigen Ausrichtung. Gleichzeitig zeigt der zweite Rückspeisekanal eine leichte Zunahme im Ausgangssignal. Dieses Verhalten war auch für negative Winkel symmetrisch zu beobachten. Die Elektroden mit dem Durchmesser von 5 mm erzeugten ein maximales Verhältnis zwischen Ausgang und Eingang von etwa 40 %, während die Elektroden mit dem Durchmesser von 10 mm einen Maximalwert von beinahe 70% Prozent erreichten.

Es kann gemäß der vorliegenden Erfindung außerdem vorgesehen sein, einen bestimmten Schwellenwert vorzugeben, den das Messsignal (Ausgang/Eingang) überschreiten muss, damit die Anordnung als ausreichend justiert betrachtet wird. Im Fall, dass der Wert unterschritten wird, kann ein Warnsignal erzeugt werden.

Die verwendete Messfrequenz betrug bei diesen Versuchen 1 MHz. Allerdings wird nicht erwartet, dass die Frequenz signifikant in die Messergebnisse mit eingeht und es können alle geeigneten Frequenzbereiche für die Anregungssignale verwendet werden. Die besten Ergebnisse werden mit Frequenzen zwischen 1 kHz bis 1 MHz erwartet.

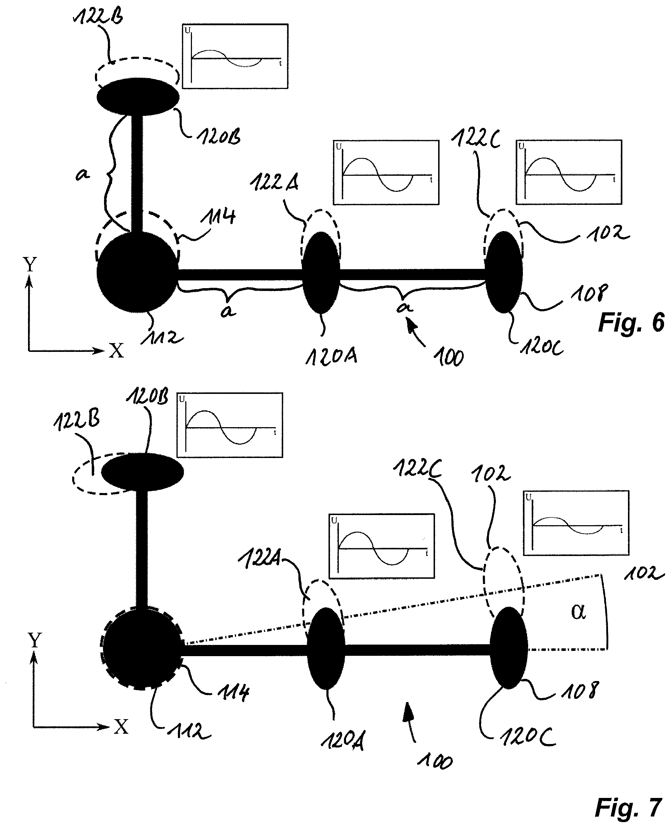

Mit Bezug auf die Figuren 6 und 7 wird nachfolgend das Messprinzip einer Justierhilfe 100 gemäß einer weiteren vorteilhaften Ausführungsform erläutert. Dabei zeigt Figur 6 die Anordnung bei einer linearen Verschiebung (hier beispielhaft in Y-Richtung), während Figur 7 eine Drehung um den Winkel α um eine Drehachse zeigt, die mit der Mitte der Anregungselektrode 112 zusammenfällt.

Die Justierhilfe 100 weist eine erste Justierelektrodenanordnung 102 auf und eine zweite Justierelektrodenanordnung 108. Beispielsweise ist die zweite Justierelektrodenanordnung 108 implantiert. Gemäß der gezeigten Ausführungsform ist neben der Anregungselektrode 112 und den beiden Auswerteelektroden 122A und 122B noch eine dritte Auswerteelektroden 122C sowie eine korrespondierende dritte Sendeelektrode 120C vorgesehen. Die Auswerteelektroden sind äquidistanten mit einem Abstand a auf gedachten Verbindungslinien, die sich in der Anregungselektrode 112 bzw. der Empfangselektrode 114 schneiden, angeordnet.

Wie in Figur 6 gezeigt, haben die Anregungselektrode 112 und die korrespondierende Empfangselektrode 114 vorzugsweise eine kreisförmige Gestalt. Die Sendeelektroden 120A bis 120C und die Auswerteelektroden 122A bis 122C haben vorzugsweise eine elliptische Gestalt. Beispielsweise kann die lange Achse der Ellipse 10 mm messen, während die kurze Achse 5 mm misst. Auf diese Weise kann zwischen einer Verschiebung in X-Richtung und einer Verschiebung in Y-Richtung unterschieden werden.

Mit dem gezeigten Layout ist es auch möglich, den Drehwinkel α zu bestimmen. Dies liegt daran, dass das Ausgangssignal, das an der Auswerteelektrode 122C abgegriffen werden kann, schneller abfällt als das Ausgangssignal, das man an den Auswerteelektroden 122A und 122B detektieren kann.

Es ist aber selbstverständlich klar, dass die oben genannten Abmessungen und speziellen Werte nur beispielhaft sind und jede andere geeignete Geometrie und Abmessung der Elektroden ebenfalls, abhängig vom Einsatzbereich, verwendet werden kann.

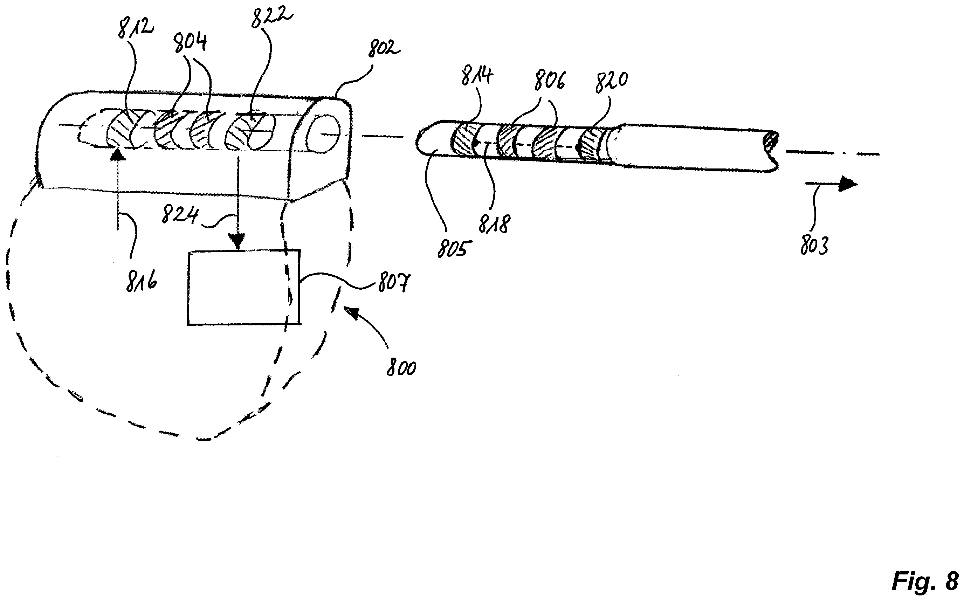

Figur 8 zeigt einen mehrkanaligen Steckverbinder für ein Stimulationsimplantat 800, bei dem die Justierhilfe gemäß der vorliegenden Erfindung ebenfalls vorteilhaft eingesetzt werden kann. Beispielsweise kann die Justierhilfe mit einem Herzschrittmacher verwendet werden, aber auch mit jeglichen anderen implantierbaren Systemen, die eine Stimulationselektrode (ohne Recording) aufweisen und ansteuern, wie beispielsweise Tiefenhirnstimulatoren, Vagusnervstimulatoren, peripheren Nervenstimulatoren usw. Wie dies allgemein bekannt ist, hat das Stimulationsimplantat 800 üblicherweise einen sogenannten Header 802, in dem die elektrischen Anschlüsse 804 für die Stimulationselektrode angeordnet sind. Gemäß der vorliegenden Erfindung kann man bei dieser Koaxialverbinderanordnung zusätzlich zu den Anschlüssen 804 eine Anregungselektrode 812 und mindestens eine Auswerteelektroden 822 vorsehen.

Gemäß den Prinzipien der vorliegenden Erfindung ist an dem Steckverbinder 805, der, wie durch den Pfeil 803 symbolisiert, zu der Stimulationselektrode führt (nicht in den Figuren gezeigt) zusätzlich zu den Gegenkontakten 806 eine Empfangselektrode 814 und eine mit ihr verbundene Sendeelektrode 820 vorgesehen. Die elektrische Verbindung 818 erfolgt vorzugsweise im Inneren des Stimulationselektrodenanschlusses 805.

Wie durch den Pfeil 816 symbolisiert, wird die Anregungselektrode 812 mit einem Anregungssignal beaufschlagt, das im zusammengesteckten Zustand von der Empfangselektrode 814 empfangen wird. Über die elektrische Verbindung 818 wird das Signal an die Sendeelektrode 820 übertragen und auf kapazitivem Wege an die Auswerteelektrode 822 gekoppelt.

Das Stimulationsimplantat 800 kann entsprechend ein Messsignal 824 auslesen, wenn der Steckverbinder 805 zufriedenstellend an dem Header 802 justiert ist. Im Fall, dass sich die Verbindung gelöst hat, fällt das Messsignal 824 unter einen spezifizierten Schwellenwert. Das Stimulationsimplantat 800 kann dann beispielsweise ein Alarmsignal erzeugen und ausgeben.

Mit der in Figur 8 gezeigt Anordnung kann in vorteilhafter Weise überwacht werden, ob eine Stimulationselektrode mit einem aktiven implantierten Stimulationsimplantat korrekt verbunden ist. Im Falle, dass sich der Steckverbinder 805 aus dem Header 802 gelöst hat, kann eine in dem Stimulationsimplantat enthaltene Steuer und Auswerteelektronik 807 ein Alarmsignal erzeugen und ausgeben. Ein potentielles Versagen kann daher rechtzeitig erkannt werden.

Zusammenfassend ist die vorliegende Erfindung in der Lage, ein robustes und präzises System zur Überwachung einer korrekten Justierung bereitzustellen, mit dem lineare und rotatorische Fehljustierung zweier einander gegenüberliegend angeordneter Elektrodenanordnungen detektiert werden können. Da die implantierten Strukturen gemäß der vorliegenden Erfindung als Transceiver fungieren, kann die Anregung und Auswertung außerhalb des Körpers vorgenommen werden, so dass das erfindungsgemäße System in jeder Anordnung integriert werden kann, in der eine direkte Rückkopplung nicht möglich ist. Darüber hinaus ist die vorliegende Erfindung natürlich nicht auf die Justierung von externen zu implantierten Elektrodenarrays beschränkt, sondern kann überall dort gewendet werden, wo eine Justierung überwacht werden muss, und nur einer der beiden Partner unmittelbar elektrisch kontaktiert werden kann.

**Bezugszeichenliste:**

| **Bezugsziffer** | **Beschreibung** |
|---|---|
| 100 | Justierhilfe |
| 102 | Erste Justierelektrodenanordnung |
| 104 | Außenseite |
| 106 | Haut |
| 108 | Zweite Justierelektrodenanordnung |
| 110 | Innenseite |
| 112, 812 | Anregungselektrode |
| 114, 814 | Empfangselektrode |
| 116, 816 | Anregungssignal |
| 118, 118A, 118B, 818 | Elektrische Verbindungen |
| 120, 120A, 120B, 1200, 820 | Sendeelektrode |
| 122, 122A, 122B, 122C, 822 | Auswerteelektrode |
| 124, 124A, 124B, 824 | Messsignal |
| 126 | Verbinder |
| 128 | Gegenverbinder |
| 130 | Erste Kopplungselektrode |
| 132 | Zweite Kopplungselektrode |
| 134 | Substrat |
| 136 | Substrat |
| 138 | Verbinderanordnung |
| 140 | Anschlussbereich |
| 142 | Anschlussbereich |
| 401-402 | Messkurven |
| 501-504 | Messkurven |
| 505 | Drehpunkt |
| 800 | Stimulationsimplantat |
| 803 | Zuleitung zur Stimulationselektrode |
| 804 | Anschlüsse für Stimulationselektrode |
| 805 | Steckverbinder |
| 806 | Gegenkontakte |
| 807 | Steuer- und Auswerteeinheit |

## Patentansprüche

1. Drahtlose Verbinderanordnung mit mindestens einer ersten und einer zweiten Komponente und mit einer Justierhilfe (100), wobei mindestens die zweite Komponente implantierbar ist, wobei die Justierhilfe (100) zum Ausrichten der mindestens einen ersten und der zweiten Komponente mit Bezug aufeinander aufweist:
eine erste Justierelektrodenanordnung (102), die mindestens eine Anregungselektrode (112) und mindestens eine Auswerteelektrode (122) aufweist und an der ersten Komponente angeordnet ist,
eine zweite Justierelektrodenanordnung (108), die mindestens eine Empfangselektrode (114) und mindestens eine Sendeelektrode (120) aufweist und an der zweiten Komponente angeordnet ist,
eine Steuer- und Auswerteschaltung, die mit der ersten Justierelektrodenanordnung (102) verbunden ist, wobei die Steuer- und Auswerteschaltung betrieben werden kann, die Anregungselektrode (112) mit einer Anregungsspannung (116) zu speisen und an der Auswerteelektrode (122) ein Messsignal (124) abzugreifen, das von einem Überdeckungsgrad der ersten und zweiten Justierelektrodenanordnung abhängt.

2. Drahtlose Verbinderanordnung nach Anspruch 1, wobei im optimal justierten Zustand der ersten und zweiten Komponente mit Bezug aufeinander die Anregungselektrode (112) und die Empfangselektrode (114) einander überdeckend ausgerichtet sind, um einen Speisekondensator auszubilden, und die Sendeelektrode (120) und die Auswerteelektrode (122) einander überdeckend ausgerichtet sind, um einen Messkondensator auszubilden.

3. Drahtlose Verbinderanordnung nach Anspruch 1 oder 2, wobei die mindestens eine Empfangselektrode (114) und die mindestens eine Sendeelektrode (120) elektrisch leitend miteinander verbunden sind.

4. Drahtlose Verbinderanordnung nach einem der vorhergehenden Ansprüche, wobei die erste Justierelektrodenanordnung (102) ein erstes planares Elektrodenarray aufweist, und die zweite Justierelektrodenanordnung (108) ein zweites planares Elektrodenarray aufweist, das in einer Ebene parallel zu dem ersten planaren Elektrodenarray angeordnet ist, wenn die erste und zweite Komponente mit Bezug aufeinander justiert sind.

5. Drahtlose Verbinderanordnung nach einem der vorhergehenden Ansprüche, wobei die erste Justierelektrodenanordnung (102) zwei Auswerteelektroden (122A, 122B) aufweist, die so angeordnet sind, dass ihre Verbindungslinien mit der Anregungselektrode (112) einander unter einem Winkel verschieden von 0° schneiden, und wobei die zweite Justierelektrodenanordnung (108) zwei Sendeelektroden (120A, 120B) aufweist, die so angeordnet sind, dass ihre Verbindungslinien mit der Empfangselektrode (114) einander unter einem Winkel verschieden von 0° schneiden.

6. Drahtlose Verbinderanordnung nach Anspruch 5, weiterhin umfassend eine dritte Auswerteelektrode (120C), die auf der Verbindungslinie der zweiten Auswerteelektrode und der Anregungselektrode angeordnet ist, wobei die zweite Justierelektrodenanordnung (102) eine korrespondierende dritte Sendeelektrode (120C) aufweist, die auf der Verbindungslinie zwischen der zweiten Sendeelektrode und der Empfangselektrode angeordnet ist.

7. Drahtlose Verbinderanordnung nach Anspruch 6, wobei die Anregungselektrode (112) und die Auswerteelektroden (122) sowie die Empfangselektrode (114) und die Sendeelektroden (120) äquidistant angeordnet sind.

8. Drahtlose Verbinderanordnung nach einem der vorhergehenden Ansprüche, wobei die Anregungselektrode (112) und die Auswerteelektroden (122) sowie die Empfangselektrode (114) und die Sendeelektrode (120) kreisförmige und/oder elliptische und/oder polygonale Metallisierungsstrukturen aufweisen.

9. Drahtlose Verbinderanordnung nach einem der Ansprüche 1 bis 8, wobei die zweite Komponente ein implantierbares Kopplungselektrodenarray aufweist, und die erste Komponente ein externes Kopplungselektrodenarray aufweist, welches das implantierbare Kopplungselektrodenarray im implantierten Zustand elektrisch kontaktiert.

10. Drahtlose Verbinderanordnung nach einem der Ansprüche 1 bis 8, wobei die erste Komponente einen implantierbaren Koaxialsteckverbinder aufweist und die zweite Komponente einen implantierbaren Koaxialgegensteckverbinder aufweist.

11. Drahtlose Verbinderanordnung nach Anspruch 10, wobei die erste Komponente eine Koaxialbuchse (802) eines Stimulationsimplantats (800) ist und die zweite Komponente der Koaxialstecker (805) einer Stimulationselektrode ist.

12. Verfahren zum Überwachen der Position einer ersten Komponente und einer zweiten Komponente mit Bezug aufeinander, wobei das Verfahren eine drahtlose Verbinderanordnung nach einem der Ansprüche 1 bis 8 verwendet, die erste Komponente nicht implantierbar ist und das Verfahren die folgenden Schritte aufweist:
Anlegen einer Anregungsspannung (116) an die mindestens eine Anregungselektrode (112),
Abgreifen einer Messspannung (124) an der mindestens einen Auswerteelektrode (122),
Vergleichen der Messspannung (124) mit einem voreingestellten Schwellenwert,
Verändern der Position der ersten Komponente in Bezug auf die zweite Komponente, bis die Messspannung (124) den voreingestellten Schwellenwert übersteigt.

13. Verfahren nach Anspruch 12, wobei die Steuer- und Auswerteschaltung ein Warnsignal erzeugt, wenn die Messspannung (124) unterhalb des voreingestellten Schwellenwerts liegt.

## Claims

1. Wireless connector assembly with at least a first and a second component and with an adjustment aid (100), wherein at least said second component is implantable, wherein the adjustment aid (100) comprises for aligning the at least one first and the second component relative to one another:
a first adjustment electrode assembly (102) which comprises at least one excitation electrode (112) and at least one evaluation electrode (122) and is arranged on said first component,
a second adjustment electrode assembly (108) which comprises at least one reception electrode (114) and at least one transmission electrode (120) and is arranged on said second component,
a control and evaluation circuit which is connected to said first adjustment electrode assembly (102), wherein said control and evaluation circuit can be operated to supply said excitation electrode (112) with an excitation voltage (116) and to tap a measurement signal (124) at said evaluation electrode (122), said measurement signal depending on a degree of overlap between said first and said second adjustment electrode assembly.

2. Wireless connector assembly according to claim 1, where, in the optimally adjusted state of said first and said second component relative to one another, said excitation electrode (112) and said reception electrode (114) are aligned to overlap one another to form a feed capacitor, and said transmission electrode (120) and said evaluation electrode (122) are aligned to overlap one another to form a measuring capacitor.

3. Wireless connector assembly according to claim 1 or 2, wherein said at least one reception electrode (114) and said at least one transmission electrode (120) are connected to one another in an electrically conductive manner.

4. Wireless connector assembly according to one of the preceding claims, wherein said first adjustment electrode assembly (102) comprises a first planar array of electrodes and said second adjustment electrode assembly (108) comprises a second planar array of electrodes which is arranged in a plane parallel to said first planar array of electrodes when said first and said second component are adjusted relative to one another.

5. Wireless connector assembly according to one of the preceding claims, wherein said first adjustment electrode assembly (102) comprises two evaluation electrodes (122A, 122B) which are arranged such that their connecting lines to said excitation electrode (112) intersect at an angle different from 0°, and wherein said second adjustment electrode assembly (108) comprises two transmission electrodes (120A, 120B) that are arranged such that their connecting lines to said reception electrode (114) intersect at an angle different from 0°.

6. Wireless connector assembly according to claim 5, further comprising a third evaluation electrode (120C) which is arranged on the connecting line of said second evaluation electrode and said excitation electrode, wherein said second adjustment electrode assembly (102) comprises a corresponding third transmission electrode (120C) which is arranged on the connection line between said second transmission electrode and said reception electrode.

7. Wireless connector assembly according to claim 6, wherein said excitation electrode (112) and said evaluation electrodes (122) as well as said reception electrode (114) and said transmission electrodes (120) are arranged equidistantly.

8. Wireless connector assembly according to one of the preceding claims, wherein said excitation electrode (112) and said evaluation electrodes (122) as well as said reception electrode (114) and said transmission electrode (120) have circular and/or elliptical and/or polygonal metallization structures.

9. Wireless connector assembly according to one of the claims 1 to 8, wherein said second component comprises an implantable array of coupling electrodes and said first component comprises an external array of coupling electrodes which establishes electrical contact to said implantable array of coupling electrodes in the implanted state.

10. Wireless connector assembly according to one of the claims 1 to 8, wherein said first component comprises an implantable coaxial plug connector and said second component comprises an implantable coaxial mating plug connector.

11. Wireless connector assembly according to claim 10, wherein said first component is a coaxial socket (802) of a stimulation implant (800) and said second component is the coaxial plug connector (805) of a stimulation electrode.

12. Method for monitoring the position of a first component and a second component relative to one another, wherein the method uses a wireless connector assembly according to one of the claims 1 to 8, wherein the first component is not implantable, and wherein the method comprises the following steps:
applying an excitation voltage (116) to said at least one excitation electrode (112),
tapping a measurement voltage (124) at said at least one evaluation electrode (122),
comparing said measurement voltage (124) with a preset threshold value;
changing the position of said first component with respect to said second component until said measurement voltage (124) exceeds said preset threshold value.

13. Method according to claim 12, wherein said control and evaluation circuit generates a warning signal when said measurement voltage (124) is below said preset threshold value.

## Revendications

1. Agencement de connecteurs sans fil comprenant au moins des premier et second composants et une aide au réglage (100), dans lequel au moins le second composant peut être implanté, dans lequel l'aide au réglage (100) pour orienter les au moins un premier et second composants l'un par rapport à l'autre comprend :
un premier agencement d'électrodes de réglage (102), qui présente au moins une électrode d'excitation (112) et au moins une électrode d'évaluation (122) et est disposé sur le premier composant,
un second agencement d'électrodes de réglage (108) qui présente au moins une électrode de réception (114) et au moins une électrode d'émission (120) et qui disposé sur le second composant,
un circuit de commande et d'évaluation, qui est connecté au premier agencement d'électrodes de réglage (102), dans lequel le circuit de commande et d'évaluation peut être actionné pour alimenter l'électrode d'excitation (112) avec une tension d'excitation (116) et pour prélever au niveau de l'électrode d'évaluation (122) un signal de mesure (124) qui dépend d'un degré de recouvrement des premier et second agencements d'électrodes de réglage.

2. Agencement de connecteurs sans fil selon la revendication 1, dans lequel, dans l'état réglé de manière optimale des premier et second composants l'un par rapport à l'autre, l'électrode d'excitation (112) et l'électrode de réception (114) sont orientées de manière à se chevaucher l'une l'autre pour former un condensateur d'alimentation, et l'électrode d'émission (120) et l'électrode d'évaluation (122) sont orientées de manière à se chevaucher l'une l'autre pour former un condensateur de mesure.

3. Agencement de connecteurs sans fil selon la revendication 1 ou 2, dans lequel la au moins une électrode de réception (114) et la au moins une électrode d'émission (120) sont connectées électriquement de manière conductrice l'une à l'autre.

4. Agencement de connecteurs sans fil selon l'une quelconque des revendications précédentes, dans lequel le premier agencement d'électrodes de réglage (102) comprend un premier réseau d'électrodes planaires et le second agencement d'électrodes de réglage (108) comprend un second réseau d'électrodes planaires agencé dans un plan parallèle au premier réseau d'électrodes planaires lorsque les premier et second composants sont réglés l'un par rapport à l'autre.

5. Agencement de connecteurs sans fil selon l'une quelconque des revendications précédentes, dans lequel le premier agencement d'électrodes de réglage (102) comprend deux électrodes d'évaluation (122A, 122B) qui sont agencées de telle sorte que leurs lignes de connexion avec l'électrode d'excitation (112) se recoupent l'une l'autre selon un angle différent de 0°, et dans lequel le second ensemble d'électrodes de réglage (108) comprend deux électrodes d'émission (120A, 120B) agencées de telle sorte que leurs lignes de connexion avec l'électrode de réception (114) se recoupent l'une l'autre selon un angle différent de 0°.

6. Agencement de connecteurs sans fil selon la revendication 5, comprenant en outre une troisième électrode d'évaluation (120C) agencée sur la ligne de connexion de la deuxième électrode d'évaluation et de l'électrode d'excitation, dans lequel le second agencement d'électrodes de réglage (102) comprend une troisième électrode d'émission correspondante (120C) agencée sur la ligne de connexion entre la deuxième électrode d'émission et l'électrode de réception.

7. Agencement de connecteurs sans fil selon la revendication 6, dans lequel l'électrode d'excitation (112) et les électrodes d'évaluation (122) ainsi que l'électrode de réception (114) et les électrodes d'émission (120) sont agencées de manière équidistante.

8. Agencement de connecteurs sans fil selon l'une quelconque des revendications précédentes, dans lequel l'électrode d'excitation (112) et les électrodes d'évaluation (122) ainsi que l'électrode de réception (114) et l'électrode d'émission (120) présentent des structures de métallisation circulaires et/ou elliptiques et/ou polygonales.

9. Agencement de connecteurs sans fil selon l'une quelconque des revendications 1 à 8, dans lequel le second composant comprend un réseau d'électrodes de couplage implantable, et le premier composant comprend un réseau d'électrodes de couplage externe qui met en contact électriquement le réseau d'électrodes de couplage implantable à l'état implanté.

10. Agencement de connecteurs sans fil selon l'une quelconque des revendications 1 à 8, dans lequel le premier composant comprend un connecteur coaxial implantable et le second composant comprend un connecteur coaxial implantable.

11. Agencement de connecteurs sans fil selon la revendication 10, dans lequel le premier composant est une douille coaxiale (802) d'un implant de stimulation (800) et le second composant est le connecteur coaxial (805) d'une électrode de stimulation.

12. Procédé de surveillance de la position d'un premier composant et d'un second composant l'un par rapport à l'autre, le procédé utilisant un agencement de connecteurs sans fil selon l'une quelconque des revendications 1 à 8, le premier composant n'étant pas implantable, le procédé comprenant les étapes consistant à :
appliquer une tension d'excitation (116) à la au moins une électrode d'excitation (112), prélever une tension de mesure (124) au niveau de la au moins une électrode d'évaluation (122),
comparer la tension de mesure (124) avec une valeur de seuil préréglée,
modifier la position du premier composant par rapport au second composant jusqu'à ce que la tension de mesure (124) dépasse la valeur de seuil prédéfinie.

13. Procédé selon la revendication 12, dans lequel le circuit de commande et d'évaluation génère un signal d'avertissement lorsque la tension de mesure (124) est inférieure à la valeur de seuil prédéfinie.
